# FASCICULE DE BREVET EUROPEEN

(11) **EP 3 595 904 B1**
(45) Date de publication et mention de la délivrance du brevet: **05.05.2021**
(21) Numéro de dépôt: 18712974.7
(22) Date de dépôt: 08.03.2018
(51) Int. Cl.: B41J 2/44, B41J 2/45, G02B 21/32, G02B 21/08

(54) **ÉQUIPEMENT ET PROCÉDÉ POUR LE DÉPÔT DE PARTICULES SUR UNE CIBLE**
VORRICHTUNG UND VERFAHREN ZUR ABSETZUNG VON PARTIKELN AUF EINER SCHEIBE
APPARATUS AND METHOD FOR DEPOSITING PARTICLES ON A TARGET

(30) Priorité: 15.03.2017 FR 1752128
(43) Date de publication de la demande: 22.01.2020
(73) Titulaire: Université de Bordeaux, 33000 Bordeaux (FR); Institut National de la Santé et de la Recherche Médicale (INSERM), 75013 Paris (FR)
(72) Inventeur: VIELLEROBE, Bertrand, 33700 Merignac (FR); VAUCELLE, Romain, 33000 Bordeaux (FR); GUILLEMOT, Fabien, 33210 Preignac (FR)
(74) Mandataire: IP Trust
(86) Numéro de dépôt international: PCT/FR2018/050532
(87) Numéro de publication internationale: WO 2018/167399

(56) Documents cités:
- WO-A2-03/056320
- US-A1- 2015 224 291
- J.A. BARRON ET AL: "Biological Laser Printing: A Novel Technique for Creating Heterogeneous 3-dimensional Cell Patterns", BIOMEDICAL MICRODEVICES, vol. 6, no. 2, 1 juin 2004 (2004-06-01), pages 139-147, XP055430666, NL ISSN: 1387-2176, DOI: 10.1023/B:BMMD.0000031751.67267.9f cité dans la demande

## Description

### Domaine de l'invention

La présente invention concerne le domaine de la bioimpression par laser par un procédé de transfert assisté par ordinateur pour la modélisation et l'assemblage de matériaux vivants et optionnellement non-vivants avec une organisation prescrite 2D ou 3D dans le but de produire des structures de bioingénierie servant en médecine régénérative, en pharmacologie et pour des études de biologie cellulaire.

La bioimpression assistée par laser permet d'organiser avec une haute précision les éléments individuels du tissu pendant sa fabrication via le dépôt couche après couche de cellules et de biomatériaux. Elle permet de reproduire des tissus en 3D avec une géométrie spécifique. L'approche « bottom-up », basée sur l'assemblage brique par brique puis couche par couche d'un objet, est compatible avec une automatisation du procédé de fabrication du tissu et peut fonctionner dans un environnement stérile. De plus, l'automatisation pourrait permettre de réduire les coûts, d'améliorer la qualité et la reproductibilité de la fabrication de tissus biologiques.

L'invention concerne plus particulièrement une solution de dépôt assisté par laser basée sur l'action directe (absorption de la radiation laser) et indirecte (création d'un plasma et d'une bulle de cavitation) d'un faisceau laser pour diriger le dépôt de particules vers un substrat d'impression avec une résolution micrométrique. Ce procédé a été utilisé pour déposer des cellules embryonnaires de la moelle épinière, guidées par les forces laser jusqu'à l'intérieur d'une fibre, puis expulsées une à une de la fibre grâce à l'étroitesse de la fibre.

### Etat de la technique

On connaît dans l'état de la technique une solution désignée par le nom de « AFA-LIFT » et décrite dans l'article B. Hopp, T. Smausz, N. Kresz, N. Barna, Z. Bor, L. Kolozsvári, D. B. Chrisey, A. Szabó, and A. Nógrádi, "Survival and proliferative ability of various living cell types after laser-induced forward transfer," Tissue Eng. 11, 1817-23 (2005).

L'article « J. A. Barron, P. Wu, H. D. Ladouceur, and B. R. Ringeisen, "Biological laser printing: a novel technique for creating heterogeneous 3-dimensional cell patterns," Biomed. Microdevices 6, 139-147 (2004) » décrit également un équipement de type « AFA LIFT ou DRL-LIFT », où la direction du laser est fixe, et le film supportant les cellules à transférer est mobile.

Un autre article, publié dans le « Journal of laser micro/nanoengineering » Vol 9, N°2-2014 sous le titre « Laser tool for single cell transfer » décrit un autre exemple de mise en œuvre de procédé et d'équipement de type AFA-LIFT.

L'article « F. Guillemot, A. Souquet, S. Catros, B. Guillotin, J. Lopez, M. Faucon, B. Pippenger, R. Bareille, M. Rémy, S. Bellance, P. Chabassier, J. C. Fricain, and J. Amédée, "High- throughput laser printing of cells and biomaterials for tissue engineering," Acta Biomater. 6, 2494-2500 (2010) » décrit un exemple d'équipement pour mettre en œuvre un tel procédé.

On connaît aussi dans l'état de la technique la demande de brevet WO2016097619 décrivant un procédé et un équipement d'impression d'au moins une encre, ledit procédé comprenant une étape de focalisation d'un faisceau laser de manière à générer une cavité dans un film d'encre, une étape de formation d'au moins une gouttelette d'encre à partir d'une surface libre du film d'encre et une étape de dépôt de ladite gouttelette sur une surface de dépose d'un substrat receveur, caractérisé en ce que le faisceau laser est orienté à contresens par rapport à la force gravitationnelle, la surface libre du film étant orientée vers le haut en direction de la surface de dépose placée au-dessus du film d'encre.

Cette configuration permet notamment d'obtenir une épaisseur E pour le film d'encre sensiblement constante, tout en limitant l'apparition des phénomènes de sédimentation. De plus, elle permet d'utiliser une large gamme d'encres.

### Inconvénient de l'art antérieur

Les solutions de l'art antérieur ne permettent pas d'observer le film de bioencre transférable et de déclencher l'impulsion laser dans une même séquence, c'est-à-dire simultanément ou avec un décalage temporel suffisamment faible pour que les éléments transférables du film de bioencre n'aient pas évolué ni migré spatialement en X, Y et Z. Les solutions connues imposent de dissocier les phases d'analyse optique du film, et les phases de déclenchement de l'impulsion, ou de modifier l'angle d'observation par rapport à la direction du laser.

Le décalage temporel ou géométrique entre l'observation et le tir laser a pour conséquence défavorable le risque d'une modification de l'état du film de bioencre transférable. L'impulsion laser qui est ensuite déclenchée n'atteint alors pas de façon certaine la cible visée.

Les solutions connues ne permettent pas de réaliser une alternance de phase à des fréquences élevées, supérieures à quelques hertz, car il faut après chaque tir un temps suffisant pour replacer l'équipement en configuration d'observation.

Les solutions connues ne permettent pas non plus de procéder à une analyse optique sous le même angle que le faisceau laser avec une résolution suffisante pour la sélection d'une particule spécifique du film de bioencre juste avant le tir laser, ni d'observer simultanément le tir et l'évolution du film de bioencre transférable.

Par ailleurs, les montages optiques de l'art antérieur créent des aberrations optiques dans la zone d'observation et de focalisation.

Les solutions de l'art antérieur connue sous le nom de AFA-LIFT, permettent de combiner une observation ponctuelle de la zone du film et la focalisation d'une impulsion laser, mais pas de combiner l'observation d'une image étendue de la zone du film. Dans la solution AFA-LIFT, la direction du faisceau laser est fixe et c'est le film qui est mobile. L'observation se limite ainsi à un champ très réduit correspondant à la zone activée par le laser, et en particulier à une seule particule, et ne permet pas d'observer une zone plus étendue.

En effet, l'objectif commun à l'observation et à la focalisation laser ne peut à la fois être adapté à la focalisation très précise du laser, et en même temps présenter un champ important pour l'observation.

De ce fait, les solutions de l'art antérieur ne permettent pas d'observer une zone étendue, par exemple pour sélectionner automatiquement une particule visible dans le champ, et piloter ensuite l'orientation du faisceau laser pour activer cette particule sélectionnée.

Enfin, l'invention vise aussi à maximiser la résolution de l'image observée et à éviter toute déformation due à une erreur de parallaxe qui se produit lorsque le champ d'observation présente un angle d'incidence différent de celui du faisceau laser.

### Solution apportée par l'invention

Afin de remédier à ces inconvénients, la présente invention concerne selon son acception la plus générale un équipement pour le dépôt de particules sur une cible à partir d'une lame transparente portant un film formé par un fluide contenant des particules en suspension, par excitation locale du film par un faisceau laser orienté par un moyen de déviation optique piloté, l'équipement comportant des moyens d'observation de ladite zone d'activation locale par un système d'imagerie optique comportant un capteur et une source d'éclairage dont les axes optiques sont sensiblement communs dans la partie comprise entre un séparateur optique et ledit film, caractérisé en ce que
a) le faisceau optique du système d'imagerie et le faisceau optique du laser sont coaxiaux dans la partie comprise entre ledit moyen de déviation optique piloté et ledit film
b) l'équipement comporte un premier bloc optique de focalisation disposé entre ledit moyen de déviation optique piloté et ledit film
c) l'équipement comporte un deuxième bloc optique de conjugaison d'image placé entre ledit capteur et ledit séparateur, le capteur étant placé dans le plan focal dudit deuxième bloc optique.

On entend par « film » au sens de la présente invention une couche mince d'un substrat, généralement liquide ou colloïdal, contenant les particules à transférer sur une cible. Cette couche peut présenter une épaisseur de quelques dizaines à quelques centaines de micromètres. Elle peut aussi être constituée par une couche dans un volume plus épais de substrat, par exemple une couche intermédiaire d'une cuve contenant le substrat chargé de particules.

On entend par « particule » au sens de la présente invention une particule organique, minérale ou vivante, en particulier :
- des particules nanométriques telles que des exosomes ou autres vésicules produites par les cellules ou des nanoparticules de biomatériaux (hydroxyapatite) ou encore des nanocapsules de biomolécules tels que des facteurs de croissance
- des particules microscopiques telles que des cellules vivantes (cellules eucaryotes, cellules souches, globules,...), des microparticules de biomatériaux et des microcapsules de biomolécules
- des particules mésoscopiques telles que des sphéroïdes constitués par des amas de cellules ou de biomatériaux.

De préférence, les particules sont des particules biologiques, comprenant des cellules vivantes, des exosomes et optionnellement des biomatériaux.

On entend par « faisceau optique du système d'imagerie» le faisceau lumineux émis depuis le film observé, compris entre la surface observée du film de bioencre et le premier élément optique, qui est généralement le scanner assurant un balayage des faisceaux, ou éventuellement une lentille de collimation.

On entend par «faisceau optique du laser » le faisceau lumineux émis par le laser, compris entre la surface du film de bioencre et le premier élément optique.

On entend par « sensiblement communs » le fait que l'axe du faisceau d'imagerie et l'axe du faisceau d'éclairage sont confondus dans une position de référence du scanner, par exemple la position médiane. La direction de l'axe du faisceau d'imagerie et de l'axe du faisceau d'éclairage peuvent varier légèrement de part et d'autre de cette position de référence, d'une valeur correspondant à l'orientation imposée à l'axe d'observation par le scanner, dans les cas où l'axe d'éclairage reste fixe.

De préférence, l'ensemble optique formé par le premier bloc et le deuxième bloc est configuré pour présenter, dans la bande de longueur d'onde du moyen d'éclairage, une résolution spatiale supérieure à la résolution spatiale du seul premier bloc, dans la longueur du laser.

Avantageusement, la résolution R_{T} de l'ensemble formé par le premier bloc et le deuxième bloc est comprise entre 1 et 8 *µ*m.

Selon une variante particulière, ledit premier bloc est configuré pour former sur le film un spot laser de diamètre supérieur à R_{T}

Avantageusement, ledit premier bloc est configuré pour former sur le film un spot laser de diamètre inférieur à 100 *µ*m, et de préférence inférieur à 30 *µ*m, avec une capacité à atteindre une résolution maximale de quelques micromètres.

Selon une alternative, ledit laser émet dans une longueur d'onde ne comprenant pas le domaine visible, préférentiellement dans l'infrarouge ou l'UV.

Selon une autre alternative, ledit laser émet dans une longueur d'onde lumière visible et en ce que l'équipement comporte un filtre réjecteur placé sur le faisceau d'imagerie, avec une bande de réjection correspondant à la longueur d'onde du laser.

Selon un mode de réalisation particulier, ledit laser et la source d'éclairage sont activés simultanément pour permettre une observation directe de l'interaction entre le faisceau laser et le film.

Selon un exemple de mise en œuvre particulier, ledit film contient des cellules vivantes.

De préférence, ledit bloc optique est constitué par un objectif télécentrique.

Selon d'autres variantes :
- la distance entre le film et le bloc optique est ajustée pour modifier la taille du spot laser d'excitation en fonction du nombre de particules à transférer.
- le design optique du bloc optique comme les traitements des surfaces des lentilles le constituant permettent de prévenir toute dégradation dudit bloc optique par l'énergie du faisceau laser.
- le rapport des focales des deux blocs optiques permet de former dans le plan du capteur de la caméra une image dont les plus petits objets observés présentent une taille de plus d'un pixel.
- les trois faisceaux laser, d'imagerie et d'éclairage sont coaxiaux et superposés entre le miroir dichroïque et le film.
- le faisceau d'éclairage se trouve de l'autre côté du film dans une position coaxiale mais non superposée par rapport aux faisceaux laser et d'imagerie.

L'invention concerne aussi un procédé pour le dépôt de particules sur une cible à partir d'une lame transparente portant un film (3) formé par un fluide contenant des particules en suspension, par excitation locale du film par un faisceau laser orienté par un moyen de déviation optique piloté, et d'imagerie de la zone d'excitation locale par un système optoélectronique d'imagerie caractérisé en ce que l'on procède à l'acquisition d'une succession d'images pendant une plage de temps encadrant le déclenchement du tir laser au moyen d'un équipement comportant des moyens d'observation de ladite zone d'excitation locale par un système d'imagerie optique comportant un capteur et une source d'éclairage dont les axes optiques sont sensiblement communs dans la partie comprise entre un séparateur optique et ledit film,
a) le faisceau optique du système d'imagerie et le faisceau du laser étant coaxiaux dans la partie comprise entre ledit moyen de déviation optique piloté et ledit film
b) l'équipement comportant un premier bloc optique de focalisation disposé entre ledit moyen de déviation optique piloté et ledit film
c) l'équipement comportant un deuxième bloc optique de conjugaison d'image placé entre ledit capteur et ledit séparateur, le capteur étant placé dans le plan focal dudit deuxième bloc optique.

### Description détaillée d'un exemple non limitatif de réalisation

D'autres caractéristiques et avantages ressortiront de la description qui va suivre de l'invention, description donnée à titre d'exemple uniquement, se référant aux dessins annexés sur lesquels :
- La figure 1 représente une vue schématique d'un équipement selon une première variante de l'invention
- La figure 2 représente une vue schématique d'un équipement selon une deuxième variante de l'invention
- La figure 3 représente une vue schématique d'un équipement selon une troisième variante de l'invention
- la figure 4 représente une vue schématique détaillée du schéma optique de l'objectif et du deuxième bloc optique de conjugaison d'image
- la figure 5 représente un exemple de spot diagramme
- la figure 6 représente une vue des déformations optiques du champ observé.

### Description d'un premier exemple d'équipement

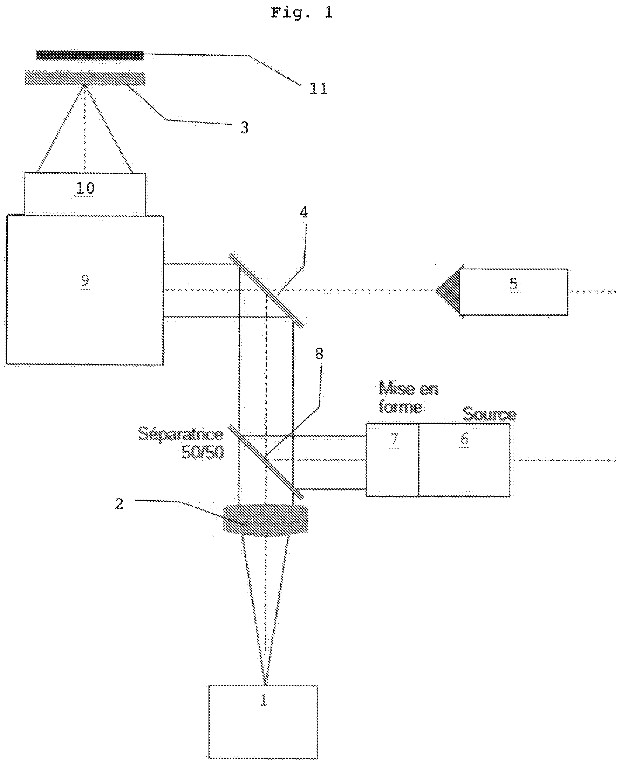

La figure 1 représente une vue schématique d'un premier exemple d'équipement.

Il comprend une caméra (1) constituée par un capteur de haute définition, par exemple de 18 millions de pixels. A titre d'exemple, la caméra (1) est un capteur commercialisé sous la référence « USB 3 uEye CP » par la société IDS.

Cette caméra (1) est associée à un deuxième bloc optique de conjugaison d'image (2) agissant comme une lentille de champ et assurant ainsi la conjugaison de l'image entre le plan focal du film (3) et le plan du capteur de la caméra (1).

Le film (3) est disposé en face d'une cible (11) vers laquelle sont transférées les cellules ou particules, lors du déclenchement d'un faisceau laser (5) impulsionnel.

A titre d'exemple, le deuxième bloc optique de conjugaison d'image (2) est constitué par un objectif, de préférence télécentrique, comprenant au moins deux lentilles optimisées dans le domaine du visible.

Le trajet optique est renvoyé par une lame dichroïque (4) passe-haut, transmettant la longueur d'onde d'émission du laser (5), par exemple l'infra-rouge et réfléchissant les longueurs d'onde dans le domaine du visible. Cette lame dichroïque (4) est orientée de façon à ce qu'à sa sortie, le faisceau du système d'imagerie (1), le faisceau d'éclairage (6) et le faisceau laser (5) soient coaxiaux et passent par le même chemin de focalisation du premier bloc optique (10).

L'éclairage peut être diffus, ou constitué par un faisceau collimaté d'éclairage. Il peut être dans le visible, ou dans une bande de longueur d'onde non visible, par exemple un éclairage infrarouge, ou ultraviolet pour l'excitation de la fluorescence. Bien entendu, le système d'imagerie est adapté à la longueur d'onde d'éclairage.

L'équipement comporte aussi une source d'éclairage (6) émettant dans le domaine du visible associée à une optique de mise en forme (7) dont la fonction est de collimater la source d'éclairage (6) si nécessaire, par exemple lorsque la source présente une divergence du faisceau émis. Cette source d'éclairage peut être une source LED unique, un composant constitué par un assemblage de diodes électroluminescentes, ou une source de lumière blanche type lampes à incandescence, lampes halogènes, laser supercontinuum... Elle peut aussi être constituée par une source de spectre étroit émettant dans les longueurs d'onde permettant l'excitation de la fluorescence de marqueurs ou de particules.

Une lame séparatrice (8) a pour fonction de superposer le chemin optique d'éclairage et le chemin optique d'imagerie.

En sortie de la lame dichroïque (4), les deux faisceaux se dirigeant vers le scanner (éclairage et laser) sont colinéaires entre eux et sont de fait aussi colinéaires avec le faisceau d'imagerie revenant du film. Ainsi, les trois faisceaux sont colinéaires entre la lame dichroïque (4) et le film d'encre (3).

Ils sont défléchis par un scanner (9) assurant une orientation pilotée par un calculateur extérieur.

Le scanner (9) assure une orientation angulaire des trois faisceaux colinéaires susvisés, selon deux axes perpendiculaires, deux d'entre eux étant balayés sur le film contenant la bio-encre, l'autre étant débalayé en un faisceau d'imagerie collimaté vers le système d'imagerie. Il est par exemple constitué par deux miroirs actionnés par un actionneur électromagnétique, par exemple un scanner commercialisé par la société SCANLAB (nom commercial) sous la référence « SCANcube 14 ».

Les trois faisceaux d'imagerie, d'éclairage et laser sont ainsi colinéaires et orientés selon une même direction en sortie du scanner (9). Ainsi, la direction d'imagerie et la direction d'éclairage suivent l'orientation du faisceau laser.

Le premier bloc optique (10) a pour fonction :
a) de transformer l'orientation angulaire en un positionnement latéral selon deux axes X, Y dans le plan du film (3),
b) de focaliser le faisceau laser et le faisceau d'éclairage dans le même plan du film (3) et
c) de collecter la lumière dans le domaine visible renvoyée par le film (3), pour former l'image de la zone d'observation du film sur le capteur de la caméra (1).

On entend par « faisceau laser » le faisceau émit par le laser.

On entend par « faisceau d'imagerie » le faisceau provenant de la zone d'observation du film et dirigé vers la caméra.

Le premier bloc optique (10) est constitué par un ensemble de lentilles formant un objectif télécentrique présentant les caractéristiques suivantes :
Dans le spectre infrarouge :
- Longueur d'onde : 1030nm
- Ouverture Numérique : > 0,10
- Distance de travail : > 10mm
- Pupille d'entrée : 12mm
- Taille de champ : > 4mm
- Seuil de dommage : > 100 J

Dans le spectre visible :
- Longueur d'onde : 450-600nm
- Ouverture Numérique : > 0,10
- Distance de travail : > 10mm
- Pupille d'entrée : 12mm
- Taille de champ :> 4mm

Dans le domaine de l'infrarouge, les surfaces des lentilles sont traitées par des couches anti-reflets pour supporter des énergies laser élevées. Cela permet d'éviter la détérioration dans le temps du premier bloc optique (10) dont le design est d'ailleurs calculé pour empêcher la création de points « chauds » laser au sein dudit premier bloc optique (10)

Le miroir dichroïque (4) empêche le retour du rayonnement infrarouge laser vers la caméra (1), lors du déclenchement d'une impulsion. Optionnellement, un filtre réjecteur infrarouge peut en outre être disposé dans le chemin optique, entre le filtre dichroïque (4) et la caméra (1).

Le rapport de la focale du deuxième bloc optique (2) et de la focale du premier bloc optique (10) est déterminé pour former dans le plan du capteur de la caméra (1) une image dont les plus petits objets observés présentent une taille de plus d'un pixel.

Typiquement, la résolution R_{T} de l'ensemble optique composé du deuxième bloc optique (2), du capteur de la caméra (1) et du premier bloc optique (10) est comprise entre 1 et 5 *µ*m.

Le premier bloc (10) est configuré pour former sur le film (3) un spot laser (en anglais « Spot Size ou Diffraction Limited ») de section supérieure à R_{T} et inférieure à 100 *µ*m.

### Variante de réalisation

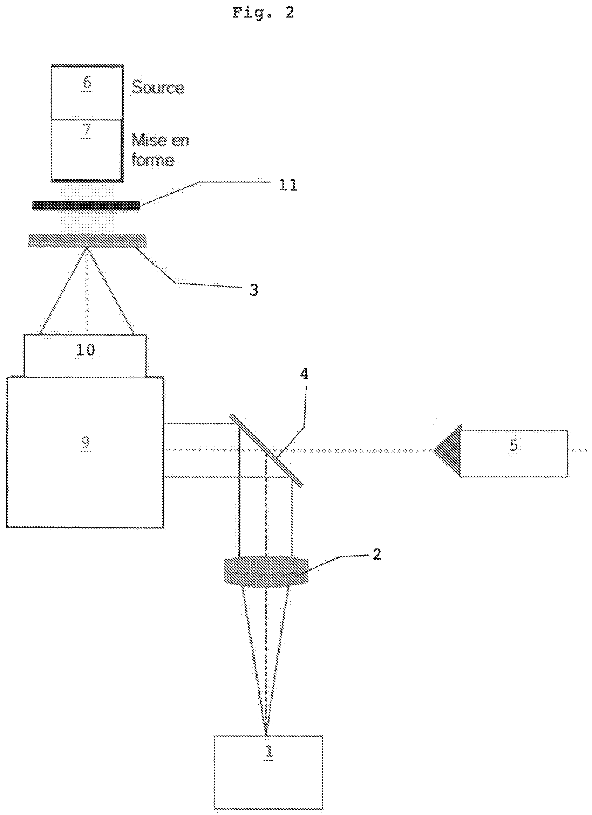

La figure 2 représente une variante de réalisation différente de celle décrite dans ce qui précède, par le fait que la source d'éclairage est sensiblement colinéaire avec le faisceau d'imagerie et le faisceau laser, mais disposée du coté opposé du film (3). Ce mode d'éclairage permet d'observer une zone de film avec un mode de contraste en transmission, et non pas en réflexion comme dans le premier mode de réalisation, intéressant pour améliorer la détection des particules dans certains types d'échantillons. Dans cette configuration, plusieurs méthodologies d'imagerie peuvent être appliquées pour améliorer ou compléter l'analyse des particules comme par exemple :
- Microcopie en transmission
- Microscopie en champ sombre (Dark field)
- Insertion de lames de phase
- Etc.

Il permet également d'éclairer l'ensemble de la zone d'observation du film de manière uniforme, et de choisir l'intervalle spectral de la source d'illumination de façon indépendante du chemin optique compris entre le miroir dichroïque (4) et le film (3).

Optionnellement, le film (3) peut être éclairé à la fois par un faisceau coaxial dans la même direction que le faisceau d'imagerie et le faisceau laser, et par un second faisceau coaxial orienté en sens opposé à la direction d'imagerie.

Dans ce cas, le dispositif est dépourvu de lame séparatrice (8).

La source d'éclairage (6) est associée à une optique de mise en forme (7) pour produire un champ d'éclairage couvrant toute la surface balayée par le scanner (9), la direction de la source d'éclairage étant fixe contrairement à la première variante de réalisation.

Cette variante implique que le substrat de la cible (11) soit transparent, pour permettre un éclairage selon une direction opposée au faisceau laser et une observation simultanée.

Si la cible (11) est opaque, il est nécessaire de prévoir un mécanisme assurant le déplacement de la cible en dehors du champ d'observation, pendant les phases d'observation. On sort alors du cadre d'observation et de tirs lasers simultanés.

### Autre variante de réalisation

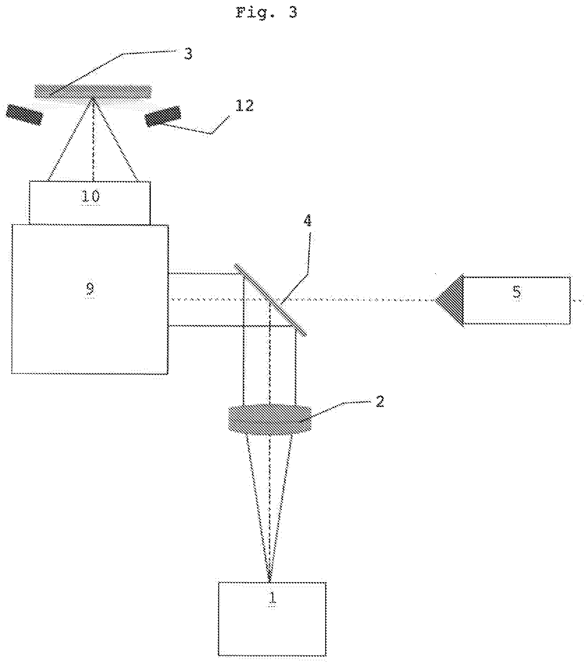

La figure 3 représente une autre alternative de réalisation, où l'éclairage est réalisé par une source annulaire (12) permettant notamment de mettre en œuvre de l'imagerie en champ sombre (dark field) en épi-illumination sur des objets opaques.

Cette source annulaire (12) est coaxiale avec l'axe médian du bloc optique (10) et produit un champ d'éclairage fixe, recouvrant l'ensemble de la zone balayée par le scanner (9).

Elle peut être disposée du même coté du bloc optique (10), par rapport au film (3), ou du coté opposé.

La source annulaire (12) permet également d'éclairer l'ensemble de la zone d'observation du film de manière uniforme, et de choisir l'intervalle spectral de la source d'illumination de façon indépendante du chemin optique compris entre le miroir dichroïque (4) et le film (3).

### Représentation 3D du schéma optique

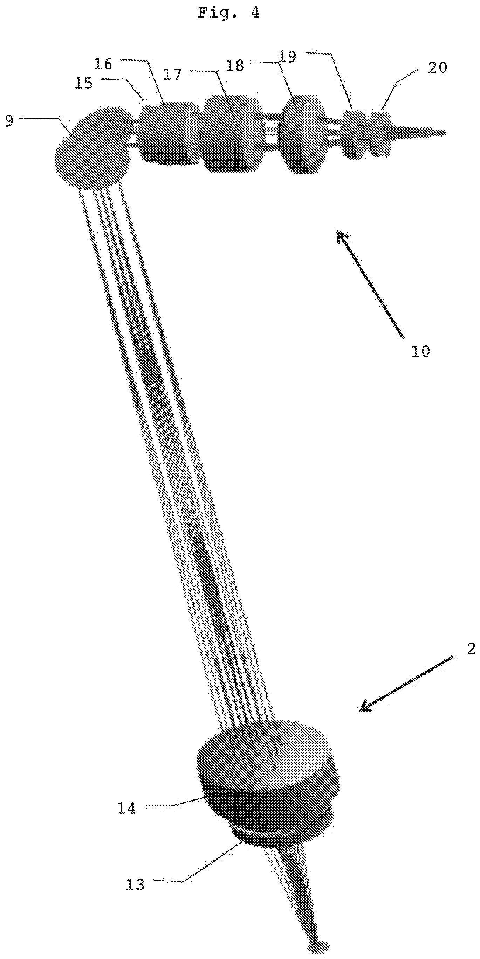

La figure 4 représente une vue détaillée du schéma optique correspondant à la première variante de réalisation.

Le deuxième bloc optique (2) est constitué de deux lentilles (13, 14).

La première lentille (13), coté caméra (1), est constituée par une lentille convexe.

La deuxième lentille (14) est constituée par un doublet formé par une lentille convexe-concave et une lentille convexe de grande focale, correspondant sensiblement à la longueur du chemin optique, typiquement de plus de 100 millimètres.

Le bloc optique (10) est constitué par un assemblage de six lentilles, avec trois lentilles d'entrée (15 à 17) convexes-concaves et trois lentilles de sortie (18 à 20) convexes.

Les designs optiques décrits ici pour chaque bloc optique (2) et (10) ne sont donnés qu'à titre indicatif comme un exemple de mise en œuvre répondant pleinement au cahier des charges décrit dans le premier mode de réalisation. Le nombre de lentilles, leurs caractéristiques et leur positionnement pourraient être différents tout en atteignant la performance recherchée. Des caractéristiques comme le prix de revient, la complexité d'intégration, la durée de vie,... ont un impact direct sur le design retenu en fonction de l'application visée.

### Forme du spot laser

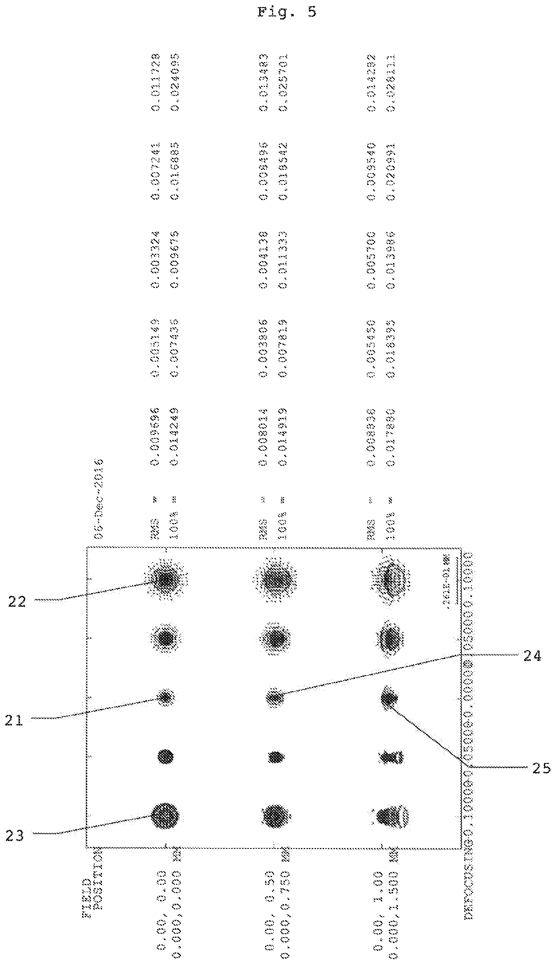

La figure 5 représente un exemple de forme du spot laser, pour différentes orientations commandées par le scanner (9) et pour différentes positions du film (3) par rapport au plan focal du bloc optique (10).

Il est à noter que le plan du film (3) n'est pas nécessairement confondu avec le plan focal du bloc optique (10), mais peut être décalé pour
- modifier la section et la forme du spot laser
- séparer le plan imagerie du plan laser pour observer un plan sur un niveau supérieur ou inférieur au plan de focalisation laser.

A cet effet, l'équipement peut disposer de moyens pour commander un déplacement de la distance relative entre le film (3) et le bloc optique (10), selon une direction perpendiculaire auxdits plans. L'effet est d'ajuster la taille du spot et de recouvrir une ou plusieurs particules, en fonction du nombre de cellules ou particules à transférer sur la cible (11).

Le spot (21) correspond à une situation où le plan focal et le plan du film (3) sont confondus, et où le spot est au centre du champ balayé par le laser (9). Dans cette situation, le spot (21) présente une section minimale, dans l'exemple décrit de 3,3 micromètres, avec des aberrations minimales. Son intensité est maximale dans cette zone centrale du champ.

Lorsque les deux plans sont décalés, on observe un élargissement du spot comme visible sur le spot (22) où la section est de 11,7 microns. Cette situation permet de transférer plusieurs particules disposées dans l'aire couverte par ce spot. La même situation est observée avec le spot (23) correspondant à un décalage des deux plans dans l'autre sens.

Lorsque l'orientation angulaire fixée par le scanner (9) est déviée par rapport à une orientation médiane, on observe un léger élargissement du spot comme visible sur les spots (24 et 25), correspondant à des orientations de part et d'autre de la direction médiane.

L'utilisation d'un objectif télécentrique a pour effet de limiter les variations de la taille du spot dues aux aberrations optiques, qui restent inférieures aux élargissements observés en cas de décalage des plans. Il permet également de ne pas tirer de façon oblique par rapport à la surface imagée comme le font les lentilles ou objectifs conventionnels, garantissant ainsi une qualité d'image dans le champ beaucoup plus homogène et reproductible.

### Variation de la taille des spots dans un même plan

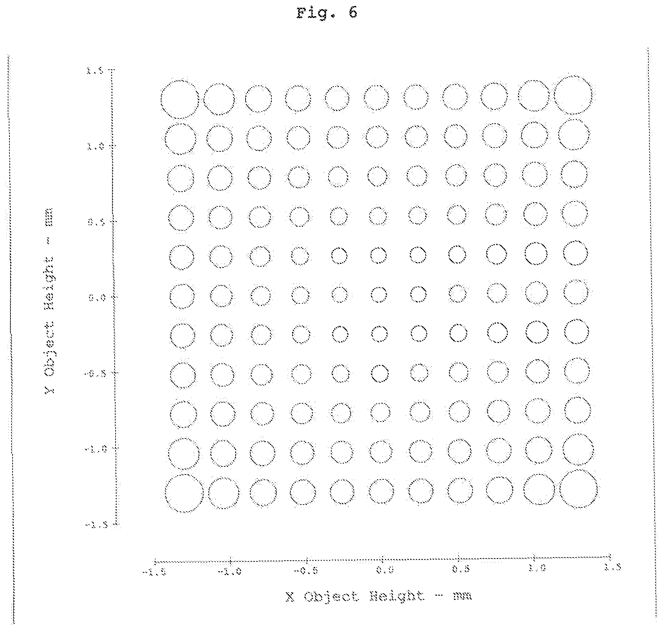

La figure 6 illustre la variation, lorsque le plan focal du bloc optique (10) est confondu avec le plan du film (3), de la taille des spots sur la surface balayée par le scanner (9) pour le design optique décrit dans la section Représentation 3D du design optique. Cette performance pourrait être sensiblement différente pour un autre design optique.

On observe une assez bonne constante dimensionnelle sur l'ensemble du champ observé de 3 millimètres sur 3 millimètres, ce qui permet de sélectionner une particule sur toute la surface observée sans dégradation significative de l'efficacité du transfert.

A titre d'exemple, la section du spot varie entre 3,3 et 8 microns selon la position dans le champ, avec une moyenne de 5 microns.

### Exemple de processus de bioimpression

Le faisceau laser étant colinéaire avec le faisceau d'illumination et le faisceau retour d'imagerie, les zones d'imagerie et de tirs lasers sont totalement superposées et liées au mouvement / position du scanner.

La séquence est la suivante :
- le scanner est positionné par rapport à une zone particulière du film de transfert et le faisceau d'éclairage vient illuminer une zone de quelques mm² afin de faire l'image du film. Généralement, cette zone contient plusieurs particules transférables, qu'il sera possible de sélectionner par le pilotage du scanner avant la séquence de tir laser
- le faisceau d'imagerie retour suit le chemin inverse jusqu'au capteur qui permet l'acquisition de l'image de la zone du film
- ensuite, un traitement informatique permet de
   - reconstruire l'image
   - corriger l'image pour la rendre plus facilement exploitable (débruitage, retrait des biais, etc.)
   - traiter l'image par des algorithmes mathématiques permettant d'analyser ses composantes principales, à savoir la détection des particules et la cartographie de leurs positions
- A partir des positions des particules est calculée une trajectoire de tirs laser permettant de faire correspondre les dits tirs lasers (et donc le nombre de particules à imprimer) avec le motif d'impression recherché (provenant de la CAO de l'objet que l'on cherche à imprimer).

La colinéarité de l'imagerie et des tirs, assurée par l'objectif unique, permet à tout moment de suivre le processus d'impression et de l'adapter en fonction des mouvements naturels (environnement) ou induits (tirs laser) du film. Ainsi, le motif d'impression est optimisé en temps réel afin d'obtenir une corrélation maximale entre le fichier d'impression calculé (en amont) et l'impression réelle, chaque tir laser correspondant à une quantité connue de particules ou de particules à déposer.

Optionnellement, le système d'imagerie permet aussi d'observer la cible et de suivre le processus de dépôt de particules sur la cible. Dans ce cas, on modifie la focalisation du système optique pour observer alternativement la zone de travail et la cible.

Une autre solution consiste à prévoir un deuxième système d'imagerie pour l'observation de la cible. Ce deuxième système d'imagerie peut observer la cible selon une direction opposée à la direction du faisceau laser, lorsque la cible est transparente, ou dans la même direction, avec une bascule séquentielle des systèmes d'imagerie.

## Revendications

1. Equipement pour le dépôt de particules sur une cible à partir d'une lame transparente portant un film (3) formé par un fluide contenant lesdites particules en suspension, par excitation locale du film (3) par un faisceau laser orienté par un moyen de déviation optique piloté, l'équipement comportant des moyens d'observation de ladite zone d'excitation locale, lesdits moyens d'observation comprenant un capteur (1) et une source d'éclairage (6) dont les axes optiques sont sensiblement communs dans la partie comprise entre un séparateur optique (4) et ledit film (3), **caractérisé en ce que** :
a) le faisceau optique du système d'imagerie (1) et le faisceau optique du laser (5) sont coaxiaux dans la partie comprise entre ledit moyen de déviation optique piloté (9) et ledit film (3)
b) l'équipement comporte un premier bloc optique de focalisation (10) disposé entre ledit moyen de déviation (9) optique piloté et ledit film (3)
c) l'équipement comporte un deuxième bloc optique de conjugaison d'image (2) placé entre ledit capteur (1) et ledit séparateur (4), le capteur (1) étant placé dans le plan focal dudit deuxième bloc optique (2).

2. Equipement selon la revendication 1 **caractérisé en ce que** la résolution de l'ensemble optique formé par le premier bloc (10) et le deuxième bloc (2), dans la bande de longueur d'onde du moyen d'éclairage est supérieure à la résolution du seul premier bloc (10), dans la longueur d'onde du laser.

3. Equipement selon la revendication 1 **caractérisé en ce que** la résolution R_{T} de l'ensemble formé par le premier bloc (10) et le deuxième bloc (2) est comprise entre 1 et 8 *µ*m.

4. Equipement selon la revendication 1 **caractérisé en ce que** ledit premier bloc (10) est configuré pour former sur le film (3) un spot laser de diamètre supérieur à la résolution R_{T} de l'ensemble formé par le premier bloc (10) et le deuxième bloc (2).

5. Equipement selon la revendication précédente **caractérisé en ce que** ledit premier bloc (10) est configuré pour former sur le film (3) un spot laser de diamètre inférieur à 100 *µ*m.

6. Equipement selon la revendication 1 **caractérisé en ce que** ledit laser (5) émet dans une longueur d'onde ne comprenant pas le domaine visible.

7. Equipement selon la revendication 1 **caractérisé en ce que** ledit laser (5) émet dans une longueur d'onde lumière visible, et **en ce qu'**il comporte un filtre réjecteur placé sur le faisceau d'imagerie, avec une bande de réjection correspondant à la longueur d'onde du laser.

8. Equipement selon la revendication 1 **caractérisé en ce que** ledit laser (5) et la source d'éclairage (6) sont activés simultanément pour permettre une observation directe de l'interaction entre le faisceau laser et le film (3).

9. Equipement selon la revendication 1 **caractérisé en ce qu'**il comprend un film (3) contenant des cellules vivantes.

10. Equipement selon la revendication 1 **caractérisé en ce que** ledit bloc optique (10) est constitué par un objectif télécentrique.

11. Equipement selon la revendication 1 **caractérisé en ce que** la distance entre le film (3) et le bloc optique (10) est ajustée pour modifier la taille du spot laser d'excitation en fonction du nombre de particules à transférer.

12. Equipement selon la revendication 1 **caractérisé en ce que** le rapport des focales des deux blocs optiques (10) et (2) permet de former dans le plan du capteur de la caméra (1) une image dont les plus petits objets observés présentent une taille de plus d'un pixel.

13. Equipement selon la revendication 1 **caractérisé en ce que** les trois faisceaux laser, d'imagerie et d'éclairage sont coaxiaux et superposés entre le miroir dichroïque (4) et le film (3).

14. Equipement selon la revendication 1 **caractérisé en ce que** le faisceau d'éclairage se trouve de l'autre côté du film (3) dans une position coaxiale mais non superposée par rapport aux faisceaux laser et d'imagerie.

15. Procédé pour le dépôt de particules sur une cible à partir d'une lame transparente portant un film (3) formé par un fluide contenant lesdites particules en suspension, par excitation locale du film (3) par un faisceau laser orienté par un moyen de déviation optique piloté, et d'imagerie de la zone d'excitation locale par un système optoélectronique d'imagerie **caractérisé en ce que** l'on procède à l'acquisition d'une succession d'images pendant une plage de temps encadrant le déclenchement du tir laser au moyen d'un équipement comportant des moyens d'observation (1) de ladite zone d'excitation locale par un système d'imagerie en lumière comportant un capteur (1) et une source d'éclairage (6) dont les axes optiques sont sensiblement communs dans la partie comprise entre un séparateur optique (4) et ledit film (3),
a) le faisceau optique du système d'imagerie (1) et le faisceau optique du laser (5) étant coaxiaux dans la partie comprise entre ledit moyen de déviation optique piloté (9) et ledit film (3)
b) l'équipement comportant un premier bloc optique de focalisation (10) disposé entre ledit moyen de déviation (9) optique piloté et ledit film (3)
c) l'équipement comportant un deuxième bloc optique de conjugaison d'image (2) placé entre ledit capteur (1) et ledit séparateur (4), le capteur (1) étant placé dans le plan focal dudit deuxième bloc optique (2).

## Patentansprüche

1. Einrichtung für die Aufbringung von Partikeln auf einem Zielträger ausgehend von einem transparenten Plättchen, das einen Film (3) trägt, der von einem die Partikel in Suspension enthaltenden Fluid gebildet wird, durch lokale Anregung des Films (3) durch einen durch ein gesteuertes optisches Ablenkmittel ausgerichteten Laserstrahl, wobei die Einrichtung Mittel zum Beobachten des Bereichs der lokalen Anregung umfasst, wobei die Mittel zum Beobachten einen Sensor (1) und eine Lichtquelle (6) umfassen, deren optische Achsen in dem Abschnitt zwischen einem Strahlteiler (4) und dem Film (3) im Wesentlichen übereinstimmen, **dadurch gekennzeichnet, dass**:
a) der optische Strahl des Bildgebungssystems (1) und der optische Strahl des Lasers (5) in dem Abschnitt zwischen dem gesteuerten optischen Ablenkmittel (9) und dem Film (3) koaxial sind
b) die Einrichtung einen ersten optischen Block zum Fokussieren (10) umfasst, der zwischen dem gesteuerten optischen Ablenkmittel (9) und dem Film (3) angeordnet ist
c) die Einrichtung einen zweiten optischen Block zum Bildzusammenfügen (2) umfasst, der zwischen dem Sensor (1) und dem Teiler (4) angeordnet ist, wobei der Sensor (1) in der Brennebene des zweiten optischen Blocks (2) angeordnet ist.

2. Einrichtung nach Anspruch 1, **dadurch gekennzeichnet, dass** die Auflösung der durch den ersten Block (10) und den zweiten Block (2) gebildeten optischen Anordnung auf dem Wellenlängenband des Beleuchtungsmittels größer als die Auflösung des ersten Block (10) allein auf der Wellenlänge des Lasers ist.

3. Einrichtung nach Anspruch 1, **dadurch gekennzeichnet, dass** die Auflösung R_{T} der durch den ersten Block (10) und den zweiten Block (2) gebildeten Anordnung zwischen 1 und 8 µm liegt.

4. Einrichtung nach Anspruch 1, **dadurch gekennzeichnet, dass** der erste Block (10) so eingerichtet ist, dass auf dem Film (3) ein Laserpunkt mit einem Durchmesser gebildet wird, der größer ist als die Auflösung R_{T} der durch den ersten Block (10) und den zweiten Block (2) gebildeten Anordnung ist.

5. Einrichtung nach dem vorhergehenden Anspruch, **dadurch gekennzeichnet, dass** der erste Block (10) so eingerichtet ist, dass auf dem Film (3) ein Laserpunkt mit einem Durchmesser von weniger als 100 µm bildet wird.

6. Einrichtung nach Anspruch 1, **dadurch gekennzeichnet, dass** der Laser (5) auf einer Wellenlänge emittiert, die den sichtbaren Bereich nicht umfasst.

7. Einrichtung nach Anspruch 1, **dadurch gekennzeichnet, dass** der Laser (5) auf einer Wellenlänge des sichtbaren Lichts emittiert und dass er einen auf dem Abbildungsstrahl angeordneten Bandsperrfilter mit einem der Wellenlänge des Lasers entsprechenden Sperrband umfasst.

8. Einrichtung nach Anspruch 1, **dadurch gekennzeichnet, dass** der Laser (5) und die Lichtquelle (6) gleichzeitig aktiviert werden, um eine direkte Beobachtung der Wechselwirkung zwischen dem Laserstrahl und dem Film (3) zu ermöglichen.

9. Einrichtung nach Anspruch 1, **dadurch gekennzeichnet, dass** sie einen lebende Zellen enthaltenden Film (3) umfasst.

10. Einrichtung nach Anspruch 1, **dadurch gekennzeichnet, dass** der optische Block (10) aus einem telezentrischen Objektiv besteht.

11. Einrichtung nach Anspruch 1, **dadurch gekennzeichnet, dass** der Abstand zwischen dem Film (3) und dem optischen Block (10) angepasst wird, um die Größe des Anregungslaserpunkts in Abhängigkeit von der Anzahl der zu übertragenden Partikel abzuändern.

12. Einrichtung nach Anspruch 1, **dadurch gekennzeichnet, dass** das Verhältnis der Brennweiten der beiden optischen Blöcke (10) und (2) es ermöglicht, in der Ebene des Sensors der Kamera (1) ein Bild zu erzeugen, dessen kleinste beobachtete Objekte eine Größe von mehr als einem Pixel aufweisen.

13. Einrichtung nach Anspruch 1, **dadurch gekennzeichnet, dass** die drei Laserstrahlen zur Bildgebung und Beleuchtung koaxial sind und sich zwischen dem dichroitischen Spiegel (4) und dem Film (3) überlagern.

14. Einrichtung nach Anspruch 1, **dadurch gekennzeichnet, dass** sich der Beleuchtungsstrahl auf der anderen Seite des Films (3) in einer bezüglich der Laser- und Bildgebungsstrahlen koaxialen, aber nicht überlagerten Position befindet.

15. Verfahren zur Aufbringung von Partikeln auf einem Zielträger ausgehend von einem transparenten Plättchen, das einen Film (3) trägt, der von einem die Partikel in Suspension enthaltenden Fluid gebildet wird, durch lokale Anregung des Films (3) durch einen durch ein gesteuertes optisches Ablenkmittel ausgerichteten Laserstrahl und zur Bildgebung des Bereichs der lokalen Anregung durch ein optoelektronisches Bildgebungssystem, **dadurch gekennzeichnet, dass** während eines die Auslösung des Laserabschusses enthaltenden Zeitraums eine Reihe von Bildern mittels einer Einrichtung erfasst wird, die Mittel zur Beobachtung (1) des Bereichs der lokalen Anregung durch eine Lichtbildgebungssystem umfasst, die einen Sensor (1) und eine Lichtquelle (6) umfassen, deren optische Achsen in dem Abschnitt zwischen einem Strahlteiler (4) und dem Film (3) im Wesentlichen übereinstimmen,
a) der optische Strahl des Bildgebungssystems (1) und der optische Strahl des Lasers (5) in dem Abschnitt zwischen dem gesteuerten optischen Ablenkmittel (9) und dem Film (3) koaxial sind
b) die Einrichtung einen ersten optischen Block zum Fokussieren (10) umfasst, der zwischen dem gesteuerten optischen Ablenkmittel (9) und dem Film (3) angeordnet ist
c) die Einrichtung einen zweiten optischen Block zum Bildzusammenfügen (2) umfasst, der zwischen dem Sensor (1) und dem Teiler (4) angeordnet ist, wobei der Sensor (1) in der Brennebene des zweiten optischen Blocks (2) angeordnet ist.

## Claims

1. Apparatus for depositing particles on a target from a transparent slide carrying a film (3) that is formed by a fluid containing said particles in suspension, by locally exciting the film (3) using a laser beam directed by a controlled optical deflection means, the apparatus comprising means for observing said local excitation region, said observation means including a sensor (1) and a lighting source (6), the optical axes of which are substantially aligned in the part between an optical splitter (4) and said film (3), **characterized in that**:
a) the optical beam of the imaging system (1) and the optical beam of the laser (5) are coaxial in the part between said controlled optical deflection means (9) and said film (3),
b) the apparatus comprises a first focusing optical unit (10) arranged between said controlled optical deflection means (9) and said film (3),
c) the apparatus comprises a second image-combining optical unit (2) positioned between said sensor (1) and said splitter (4), the sensor (1) being positioned in the focal plane of said second optical unit (2).

2. Apparatus according to claim 1, **characterized in that** the resolution of the optical assembly formed by the first unit (10) and the second unit (2) in the wavelength band of the lighting means is greater than the resolution of only the first unit (10) in the wavelength of the laser.

3. Apparatus according to claim 1, **characterized in that** the resolution R_{T} of the assembly formed by the first unit (10) and the second unit (2) is between 1 and 8 *µ*m.

4. Apparatus according to claim 1, **characterized in that** said first unit (10) is designed to form, on the film (3), a laser spot having a diameter greater than the resolution R_{T} of the assembly formed by the first unit (10) and the second unit (2).

5. Apparatus according to the preceding claim, **characterized in that** said first unit (10) is designed to form, on the film (3), a laser spot having a diameter of less than 100 *µ*m.

6. Apparatus according to claim 1, **characterized in that** said laser (5) emits in a wavelength that does not include the visible range.

7. Apparatus according to claim 1, **characterized in that** said laser (5) emits in a visible light wavelength, and **in that** it comprises a rejection filter positioned on the imaging beam and having a rejection band corresponding to the wavelength of the laser.

8. Apparatus according to claim 1, **characterized in that** said laser (5) and the lighting source (6) are activated simultaneously to allow direct observation of the interaction between the laser beam and the film (3).

9. Apparatus according to claim 1, **characterized in that** it includes a film (3) containing living cells.

10. Apparatus according to claim 1, **characterized in that** said optical unit (10) consists of a telecentric lens.

11. Apparatus according to claim 1, **characterized in that** the distance between the film (3) and the optical unit (10) is adjusted to modify the size of the excitation laser spot according to the number of particles to be transferred.

12. Apparatus according to claim 1, **characterized in that** the ratio of the focal lengths of the two optical units (10) and (2) makes it possible to form, in the plane of the sensor of the camera (1), an image of which the smallest objects observed have a size of more than one pixel.

13. Apparatus according to claim 1, **characterized in that** the three laser, imaging and lighting beams are coaxial and superimposed between the dichroic mirror (4) and the film (3).

14. Apparatus according to claim 1, **characterized in that** the lighting beam is located on the other side of the film (3) in a position that is coaxial but not superimposed with respect to the laser and imaging beams.

15. Method for depositing particles on a target from a transparent slide carrying a film (3) that is formed by a fluid containing said particles in suspension, by locally exciting the film (3) using a laser beam directed by a controlled optical deflection means, and for imaging the local excitation region using an optoelectronic imaging system, **characterized in that**, during a time period surrounding the triggering of the laser shot, a succession of images is acquired by means of an apparatus comprising means (1) for observing said local excitation region using a light imaging system comprising a sensor (1) and a lighting source (6), the optical axes of which are substantially aligned in the part between an optical splitter (4) and said film (3),
a) the optical beam of the imaging system (1) and the optical beam of the laser (5) being coaxial in the part between said controlled optical deflection means (9) and said film (3),
b) the apparatus comprising a first focusing optical unit (10) arranged between said controlled optical deflection means (9) and said film (3),
c) the apparatus comprising a second image-combining optical unit (2) positioned between said sensor (1) and said splitter (4), the sensor (1) being positioned in the focal plane of said second optical unit (2).
